# EUROPEAN PATENT APPLICATION

(11) **EP 4 715 837 A2**
(43) Date of publication of application: **25.03.2026**
(21) Application number: 24811461.3
(22) Date of filing: 04.04.2024
(51) Int. Cl.: G16H 30/40, G16H 50/70, G06T 7/00, G06T 7/136, G16H 50/20, G16H 30/00, G16H 20/10, G06T 7/11

(54) **METHOD AND DEVICE FOR ANALYZING PATHOLOGY SLIDE IMAGE**

(30) Priority: 19.05.2023 KR 20230065294; 27.10.2023 KR 20230146122
(71) Applicant: Lunit Inc., Seoul 06241 (KR)
(72) Inventor: SONG, Sang Hoon, Seoul 06241 (KR); KIM, Suk Jun, Seoul 06241 (KR); OCK, Chan Young, Seoul 06241 (KR)
(74) Representative: AWA Sweden AB
(86) International application number: PCT/KR2024/095662
(87) International publication number: WO 2024/242525

(57) **Abstract**

A computing apparatus according to an aspect includes: at least one memory; and at least one processor, wherein the at least one processor is configured to identify at least one tissue area from a pathological slide image by using an artificial intelligence model, generate spatial distribution-related information regarding the at least one tissue area on the basis of an image arithmetic operation on the at least one tissue area, and calculate an evaluation index associated with treatment responsiveness, on the basis of the spatial distribution-related information.

## Description

### Technical Field

The present disclosure relates to a method and apparatus for analyzing a pathological slide image.

### Background Art

The field of digital pathology refers to a field that acquires histological information regarding corresponding patients or predicts prognosis of the patients by using whole slide images generated by scanning pathological slide images.

Recently, technologies for predicting medical information by analyzing pathological slide images through artificial intelligence models have been developed. However, there is a need for methods of analyzing pathological slide images with high accuracy and speed and various biomarkers for predicting medical information.

### Disclosure of Invention

### Technical Problem

Provided are a method and apparatus for analyzing a pathological slide image. In addition, provided is a computer-readable recording medium having recorded thereon a program for causing a computer to execute the method. The technical problems to be solved are not limited to the technical problems as described above, and other technical problems may be present.

### Solution to Problem

A computing apparatus according to an aspect includes: at least one memory; and at least one processor, wherein the at least one processor is configured to identify at least one tissue area from a pathological slide image by using an artificial intelligence model, generate spatial distribution-related information regarding the at least one tissue area on the basis of an image arithmetic operation on the at least one tissue area, and calculate an evaluation index associated with treatment responsiveness, on the basis of the spatial distribution-related information.

A method of analyzing a pathological slide image, according to another aspect, includes: identifying at least one tissue area from a pathological slide image by using an artificial intelligence model; generating spatial distribution-related information regarding the at least one tissue area, on the basis of an image arithmetic operation on the at least one tissue area; and calculating an evaluation index associated with treatment responsiveness, on the basis of the spatial distribution-related information.

A computer-readable recording medium according to another aspect includes a recording medium having recorded thereon a program for causing a computer to execute the method described above.

### Advantageous Effects of Invention

An effective treatment strategy for a tumor may be established through a biomarker based on the result of analysis of a pathological slide image. In addition, an expert may be helped prescribe more appropriate and efficient customized treatments to patients.

### Brief Description of Drawings

FIG. 1 is a view illustrating an example of a system for analyzing a pathological slide image, according to an embodiment.
FIG. 2A is a block diagram illustrating an example of a user terminal according to an embodiment.
FIG. 2B is a block diagram illustrating an example of a server according to an embodiment.
FIG. 3 is a flowchart illustrating an example of a method of analyzing a pathological slide image, according to an embodiment.
FIG. 4 is a view illustrating an example of identifying at least one tissue area from a pathological slide image by using an artificial intelligence model, according to an embodiment.
FIG. 5 is a view illustrating an example of a spatial distribution of a tissue area, according to an embodiment.
FIG. 6 is a view illustrating an example in which a cancer area and a stroma area are in contact with each other, according to an embodiment.
FIG. 7 is an example view of a plurality of sections according to an embodiment.
FIG. 8 is an example view of a pathological slide image in which a plurality of sections are displayed, according to one embodiment.
FIGS. 9A to 9C are views illustrating an example of calculating a fragmentation index, according to an embodiment.
FIG. 10 is a view illustrating another example of a system for analyzing a pathological slide image.

### Best Mode for Carrying out the Invention

A computing apparatus according to an aspect includes: at least one memory; and at least one processor, wherein the at least one processor is configured to identify at least one tissue area from a pathological slide image by using an artificial intelligence model, generate spatial distribution-related information regarding the at least one tissue area on the basis of an image arithmetic operation on the at least one tissue area, and calculate an evaluation index associated with treatment responsiveness, on the basis of the spatial distribution-related information.

### Mode for the Invention

Terms used in embodiments are selected as currently widely used general terms as possible, which may vary depending on intentions or precedents of those skilled in the art, emergence of new technologies, and the like. In addition, in certain cases, there are also terms arbitrarily selected by the applicant, and in this case, the meaning thereof will be defined in detail in the description. Therefore, the terms used herein should be defined based on the meanings of the terms and the details throughout the description, rather than the simple names of the terms.

Throughout the description, when a part includes a certain element, it means that other elements may be further included, rather than excluding the other elements, unless otherwise stated. In addition, the term, such as "~ unit" or "~ module" described herein, refers to a unit that processes at least one function or operation, which may be implemented as hardware or software, or a combination of hardware and software.

Also, although the terms, "first", "second", etc. may be used herein to describe various components, these components should not be limited by the terms. The terms may be used to distinguish one component from another component.

According to an embodiment, "a pathological slide image" may refer to an image obtained by capturing a pathological slide that is fixed and stained via a series of chemical treatment processes for a tissue or the like removed from a human body. In addition, the pathological slide image may refer to a whole slide image (WSI) including a high-resolution image of a whole slide, and may also refer to a portion of the whole slide image, for example, one or more patches. For example, the pathological slide image may refer to a digital image captured or scanned via a scanning apparatus (e.g., a digital scanner or the like), and may include information regarding a particular protein, cell, tissue and/or structure within a human body. In addition, the pathological slide image may include one or more patches, and histological information may be applied (e.g., tagged) to the one or more patches via an annotation task.

"Medical information" may refer to any medically meaningful information that may be extracted from a medical image. For example, the medical information may include at least one of an immune phenotype, a genotype, a biomarker score, tumor purity, information regarding RNA, information regarding a tumor microenvironment, and a treatment method for cancer expressed in a pathological slide image.

Also, the medical information may include an area, location, and size of a particular tissue (e.g., a cancer tissue, a cancer stromal tissue, or the like) and/or a particular cell (e.g., a tumor cell, a lymphocyte cell, a macrophage cell, an endothelial cell, a fibroblast cell, or the like) within a medical image, diagnostic information regarding cancer, information associated with the possibility of developing cancer of a subject, and/or a medical conclusion associated with cancer treatment, but is not limited thereto.

In addition, the medical information may include not only a quantified numerical value that may be obtained from a medical image, but also information obtained by visualizing the numerical value, predictive information according to the numerical value, image information, statistical information, and the like.

For example, the medical information may be provided to a user terminal or output through a display device.

Hereinafter, embodiments are described in detail with reference to the accompanying drawings. However, the embodiments may be implemented in several different forms and are not limited to examples described herein.

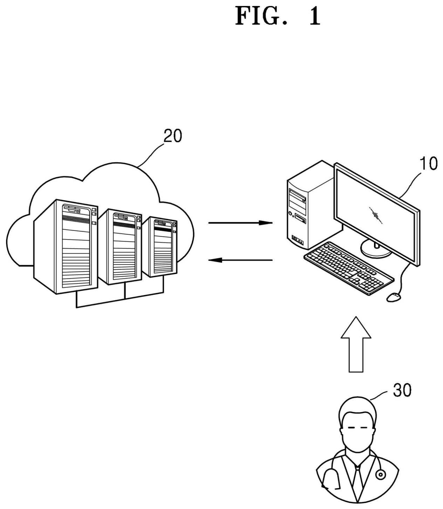

FIG. 1 is a view illustrating an example of a system for analyzing a pathological slide image, according to an embodiment.

Referring to FIG. 1, a system for analyzing a pathological slide image (hereinafter, a system) may include a user terminal 10 and a server 20. For example, the user terminal 10 and the server 20 may be connected to each other by a wired or wireless communication method to transmit and/or receive various types of data to and/or from each other.

For convenience of description, although FIG. 1 illustrates that the system includes the user terminal 10 and the server 20, the present disclosure is not limited thereto. For example, the system may include another external device (not shown). For example, operations of the user terminal 10 and the server 20 described below may be implemented by a single device (e.g., the user terminal 10 or the server 20) or more devices.

The user terminal 10 may be a computing apparatus that is provided with a display device and a device (e.g., a keyboard, a mouse, or the like) for receiving a user input, and includes a memory and a processor. Also, the display device may be implemented as a touch screen to perform a function of receiving a user input. For example, the user terminal 10 may correspond to a notebook PC, a desktop PC, a laptop, a tablet computer, a smartphone, or the like, but is not limited thereto.

The server 20 may be an apparatus that communicates with an external device (not shown) including the user terminal 10. For example, the server 20 may be an apparatus that stores various types of data including a pathological slide image, a bitmap image corresponding to the pathological slide image, information generated by analysis of the pathological slide image (e.g., information regarding at least one tissue area expressed in the pathological slide image, information regarding at least one biomarker expression, medical information related to the pathological slide image, or the like), and information regarding an artificial intelligence model used for analysis of the pathological slide image. Alternatively, the server 20 may be a computing apparatus including a memory and a processor, and having an arithmetic operation capability. In the case where the server 20 is a computing apparatus, the server 20 may perform at least some of operations of the user terminal 10 described below with reference to FIGS. 1 to 10. For example, the server 20 may also be a cloud server, but is not limited thereto.

The user terminal 10 outputs the pathological slide image and/or information generated via analysis of the pathological slide image. For example, the user terminal 10 may output various types of information regarding at least one tissue area expressed in the pathological slide image. In addition, the user terminal 10 may output expression information of a biomarker.

The pathological slide image may refer to an image obtained by capturing a pathological slide that is fixed and stained through a series of chemical treatment processes to observe a tissue or the like removed from a human body under a microscope. As an example, the pathological slide image may refer to a whole slide image including a high-resolution image for a whole slide. As another example, the pathological slide image may refer to a portion of the high-resolution whole slide image.

Meanwhile, the pathological slide image may refer to an area segmented into patch (or tile) units from the whole slide image. For example, a patch (or a tile) may have a size of a certain area.

In addition, the pathological slide image may refer to a digital image captured by using a microscope and may include information regarding a cell, a tissue, and/or a structure within a human body.

Biological factors (e.g., a cancer cell, an immune cell, a cancer area, and the like) expressed in the pathological slide image may be identified via analysis of the pathological slide image. The biological factors may be used for a histological diagnosis of a disease, prediction of a disease prognosis, a determination of a treatment direction for a disease, or the like.

The user terminal 10 may analyze the pathological slide image by using an artificial intelligence model. For example, the user terminal 10 may individually detect many cells distributed on the pathological slide image and segment each area from a tissue, by using the artificial intelligence model. In addition, the user terminal 10 may analyze the detected cells and the segmented areas and derive the result of the analysis, by using the artificial intelligence model.

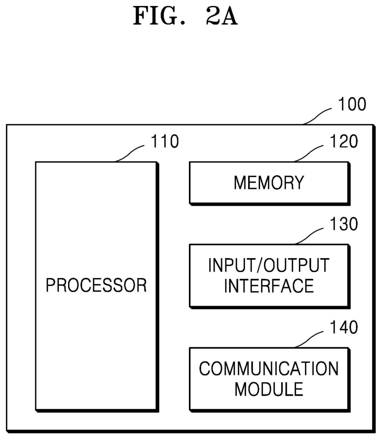

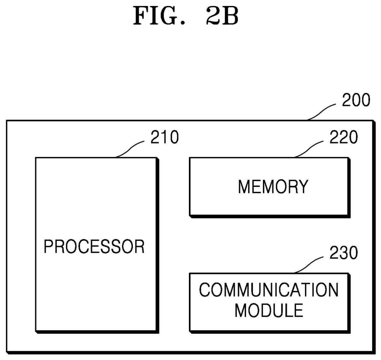

Hereinafter, an example in which the user terminal 10 analyzes a pathological slide image is described with reference to FIGS. 2 to 10.

Meanwhile, for convenience of description, the user terminal 10 has been described as performing all operations throughout the description, but is not limited thereto. For example, at least some of the operations performed by the user terminal 10 may also be performed by the server 20.

FIG. 2A is a block diagram illustrating an example of a user terminal according to an embodiment.

Referring to FIG. 2A, a user terminal 100 includes a processor 110, a memory 120, an input/output interface 130, and a communication module 140. For convenience of description, FIG. 2A illustrates only components related to the present disclosure. Accordingly, the user terminal 100 may further include other general-purpose components in addition to the components illustrated in FIG. 2A. In addition, it is obvious to those skilled in the art related to the present disclosure that the processor 110, the memory 120, the input/output interface 130, and the communication module 140 illustrated in FIG. 2A may be implemented as independent devices.

The processor 110 may process commands of a computer program by performing basic arithmetic, logic, and input/output operations. Here, the commands may be provided from the memory 120 or an external apparatus (e.g., the server 200 or the like). In addition, the processor 110 may generally control operations of other components included in the user terminal 100.

The processor 110 may identify at least one tissue area from a pathological slide image by using an artificial intelligence model. As an example, the tissue area may include a cancer area and a stroma area.

The processor 110 may generate spatial distribution-related information regarding the at least one tissue area, on the basis of an image arithmetic operation on the at least one tissue area. Here, the spatial distribution-related information may include at least one of area information of each of a plurality of sections included in the at least one tissue area based on a boundary at which the cancer area and the stroma area are in contact with each other, area information of an intersection area in which an area to which the stroma area is extended by a preset length overlaps the cancer area, and the number of fragments of the cancer area included in the at least one tissue area.

As an example, on the basis of the boundary at which the cancer area and the stroma area included in the at least one tissue area are in contact with each other, the processor 110 may segment the cancer area and the stroma area into a plurality of sections on the basis of a preset interval and calculate a density of at least one analysis target cell included in each of the plurality of sections. The processor 110 may set an analysis range in a direction of the stroma area and a direction of the cancer area because of the boundary. In addition, the processor 110 may generate the plurality of sections by segmenting the analysis range because of the interval.

In particular, on the basis of an area to which the stroma area is extended by a first length and an area to which the cancer area is contracted by the first length, the processor 110 may identify a first intersection area in which the extended area overlaps the cancer area, and determine area information regarding an area corresponding to any one section from among the plurality of sections, on the basis of the first intersection area.

In addition, on the basis of an area to which the cancer area is extended by the first length and an area to which the stroma area is contracted by the first length, the processor 110 may set an intersection area in which the extended area overlaps the stroma area, and determine area information regarding an area corresponding to one section from among the plurality of sections included in the stroma area, on the basis of the intersection area. In addition, the processor 110 may detect fragments of the cancer area.

Similarly, on the basis of an area to which the cancer area is extended by a second length greater than the first length and an area to which the cancer area is contracted by the second length, the processor 110 may identify a second intersection area in which the area extended by the second length overlaps the area to which the cancer area is contracted by the first length, and determine area information regarding an area corresponding to any one section from among the plurality of sections included in the cancer area, on the basis of the second intersection area.

The processor 110 may calculate an evaluation index associated with treatment responsiveness from at least one parameter calculated on the basis of the spatial distribution-related information. Here, the evaluation index may include at least one of a density of an analysis target cell, a contact score (CTS) (hereinafter, a tumor-stromal contact index), and a tumor fragmentation index (TFI) (hereinafter, a fragmentation index) in each of a plurality of sections generated on the basis of a tumor-stromal border (TSB) (hereinafter, a border or a tumor-stromal contact border). Hereinafter, each embodiment is described.

Subsequently, the processor 110 may calculate a density of at least one analysis target cell included in each of the plurality of sections. For example, the processor 110 may calculate a density of tumor infiltrating lymphocyte (TIL) in each of the plurality of sections.

As an example, the processor 110 may detect one or more boundary grids including a boundary, from among a plurality of grids of the pathological slide image. In addition, the processor 110 may calculate a density of a tumor infiltrating lymphocyte in each of a plurality of sections included in the one or more boundary grids.

Here, the processor 110 may predict treatment responsiveness of at least one tissue area to an immune anticancer agent on the basis of the density of the tumor infiltrating lymphocyte. In detail, the processor 110 may set a weight to a density of at least one analysis target cell in at least one section from among the plurality of sections.

As another example, as described above, the processor 110 may determine an intersection area in which an area to which the stroma area is extended by a preset length overlaps the cancer area, and calculate a tumor-stroma contact index on the basis of a ratio of an area of the intersection area to an area of the cancer area.

As another example, the processor 110 may calculate a fragmentation index on the basis of a ratio of the number of fragments of the cancer area to the area of the cancer area.

Meanwhile, the processor 110 may output the spatial distribution-related information of the at least one tissue area identified from the pathological slide image. In addition, the processor 110 may output a report including the spatial distribution-related information calculated on the basis of a spatial distribution. In addition, the processor 110 may output a report including the calculated evaluation index, on the basis of the spatial distribution-related information. For example, the processor 110 may control a display device to output at least one of the spatial distribution-related information, the evaluation index, and the report described above.

The processor 110 may be implemented as an array of a plurality of logical gates, or may be implemented as a combination of a general-purpose microprocessor and a memory that stores a program executable by the microprocessor. For example, the processor 110 may include a general-purpose processor, a central processing unit (CPU), a microprocessor, a digital signal processor (DSP), a controller, a microcontroller, a state machine, or the like. In some environments, the processor 110 may include an application-specific integrated circuit (ASIC), a programmable logic device (PLD), a field programmable gate array (FPGA), or the like. For example, the processor 110 may refer to a combination of processing devices, such as a combination of a digital signal processor (DSP) and a microprocessor, a combination of a plurality of microprocessors, a combination of one or more microprocessors combined with a digital signal processor (DSP) core, or any other combination of such components.

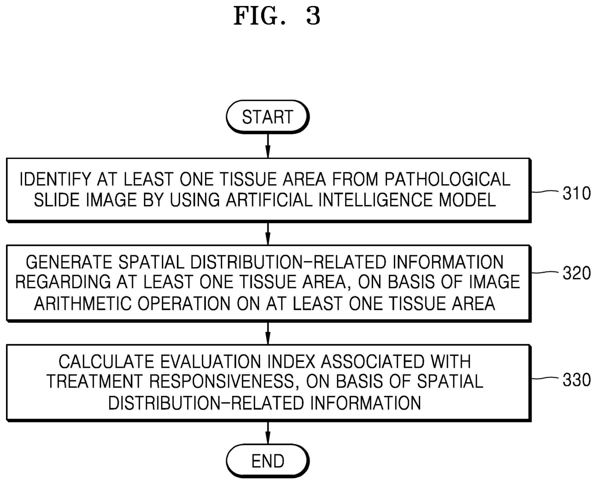

The memory 120 may include any non-transitory computer-readable recording medium. As an example, the memory 120 may include a permanent mass storage device such as random access memory (RAM), read only memory (ROM), a disk drive, a solid state drive (SSD), or flash memory. As another example, the permanent mass storage device such as ROM, an SSD, flash memory, or a disk drive may be a separate permanent storage device that is distinguished from a memory. In addition, the memory 120 may store an operating system (OS) and at least one program code (e.g., a code for the processor 110 to perform an operation described below with reference to FIGS. 3 to 10).

Software components described above may be loaded from a computer-readable recording medium that is separate from the memory 120. The separate computer-readable recording medium may be a recording medium that may be directly connected to the user terminal 100 and may include, for example, a computer-readable recording medium such as a floppy drive, a disk, a tape, a DVD/CD-ROM drive, or a memory card. Alternatively, the software components may also be loaded into the memory 120 through the communication module 140 rather than the computer-readable recording medium. For example, the at least one program may be loaded into the memory 120 on the basis of a computer program that is installed by files provided through the communication module 140 by developers or a file distribution system that distributes installation files of applications (e.g., a computer program or the like for the processor 110 to perform operations described below with reference to FIGS. 3 to 10).

The input/output interface 130 may be a unit for interfacing with a device (e.g., a keyboard, a mouse, or the like) for an input or output, which may be connected to the user terminal 100 or may be included in the user terminal 100. Although FIG. 2A illustrates the input/output interface 130 as a component configured separately from the processor 110, but the input/output interface 130 is not limited thereto and may also be configured to be included in the processor 110.

The communication module 140 may provide a component or function for the server 200 and the user terminal 100 to communicate with each other through a network. In addition, the communication module 140 may provide a component or function for the user terminal 100 to communicate with another external device. For example, a control signal, a command, data, and the like, which are provided under control of the processor 110, may be transmitted to the server 200 and/or an external device through the communication module 140 and the network.

Meanwhile, although not illustrated in FIG. 2A, the user terminal 100 may further include a display device. Alternatively, the user terminal 100 may be connected to an independent display device through a wired or wireless communication method to transmit and receive data to and from each other. For example, a pathological slide image, analysis information regarding the pathological slide image, medical information, additional information based on the medical information, and the like may be provided to a user 30 through the display device.

FIG. 2B is a block diagram illustrating an example of a server according to an embodiment.

Referring to FIG. 2B, a server 200 includes a processor 210, a memory 220, and a communication module 230. For convenience of description, FIG. 2B illustrates only components related to the present disclosure. Accordingly, the server 200 may further include other general-purpose components in addition to the components illustrated in FIG. 2B. In addition, it is obvious to those skilled in the art related to the present disclosure that the processor 210, the memory 220, and the communication module 230 illustrated in FIG. 2B may be implemented as independent devices.

The processor 210 may acquire a pathological slide image from at least one of the internal memory 220, the user terminal 100, and another external device. The processor 210 may identify at least one tissue area from the pathological slide image by using an artificial intelligence model, identify a spatial distribution of the at least one tissue area on the basis of an image arithmetic operation on the at least one tissue area, calculate an evaluation index associated with treatment responsiveness, from spatial distribution-related information calculated on the basis of the spatial distribution, or transmit, to the user terminal 100, at least one of information regarding the identified tissue area, information regarding the spatial distribution of the tissue area, the spatial distribution-related information, and the evaluation index calculated from the spatial distribution-related information. In addition, the processor 210 may transmit at least one of the evaluation index or a report (e.g., including treatment responsiveness predicted on the basis of the evaluation index) to the user terminal 100.

In other words, at least one of the operations of the processor 110 described above with reference to FIG. 2A may be performed by the processor 210. In this case, the user terminal 100 may output, through the display device, information transmitted from the server 200.

Meanwhile, an implementation example of the processor 210 is the same as the implementation example of the processor 110 described above with reference to FIG. 2A, and thus, a detailed description thereof is omitted.

The memory 220 may store various types of data such as the pathological slide image and data generated according to the operation of the processor 210. In addition, the memory 220 may store an operating system (OS) and at least one program (e.g., a program needed for the processor 210 to operate, and the like).

Meanwhile, an implementation example of the memory 220 is the same as the implementation example of the memory 120 described above with reference to FIG. 2A, and thus, a detailed description thereof is omitted.

The communication module 230 may provide a component or function for the server 200 and the user terminal 100 to communicate with each other through a network. In addition, the communication module 230 may provide a component or function for the server 200 to communicate with another external device. For example, a control signal, a command, data, and the like, which are provided under control of the processor 210, may be transmitted to the user terminal 100 and/or an external device through the communication module 230 and the network.

FIG. 3 is a flowchart illustrating an example of a method of analyzing a pathological slide image, according to an embodiment.

The method illustrated in FIG. 3 includes operations processed in a time series by the user terminal 10 or 100 or the processor 110 illustrated in FIG. 1 or 2A. Accordingly, although the above description of the user terminal 10 or 100 or the processor 110 illustrated in FIG. 1 or 2A is omitted below, the above description may also be applied to the method illustrated in FIG. 3.

In addition, as described above with reference to FIGS. 1 to 2B, at least one of the operations of the method illustrated in FIG. 3 may be processed by the server 20 or 200 or the processor 210.

In operation 310, a processor identifies at least one tissue area from a pathological slide image by using an artificial intelligence model.

In the present disclosure, the pathological slide image may be a whole slide image or a portion of the whole slide image. Here, the portion may be referred to as a patch or a tile.

In an embodiment, the pathological slide image may be divided into a plurality of grids. As an example, the processor may generate the plurality of grids by dividing the pathological slide image. Here, the plurality of grids may be formed in a grid shape. The processor may efficiently organize the pathological slide image, and easily manage and analyze data regarding each grid area by generating the plurality of grids for the pathological slide image.

In an embodiment, the processor may identify a cancer area, a stroma area, a necrosis area, a background area, and the like from the pathological slide image by analyzing the pathological slide image. In addition, the processor may classify a plurality of cells expressed in the pathological slide image into at least one of a tumor cell, a lymphocyte cell, and other cells. Here, the lymphocyte cell may include a tumor infiltrating lymphocyte cell. The processor may determine areas for the identified cancer area, stroma area, necrosis area, and background area. In addition, the processor may determine the number of the plurality of cells.

In addition, expression information of a biomarker may include the number of positive (+)/negative (-) tumor cells for programmed death-ligand 1 (PD-L1), the number of positive (+)/negative (-) immune cells, a tumor proportion score (TPS), a combined positive score (CPS), and the like. However, the expression information of the biomarker is not limited to the above description, and an evaluation index associated with treatment responsiveness may include the expression information of the biomarker.

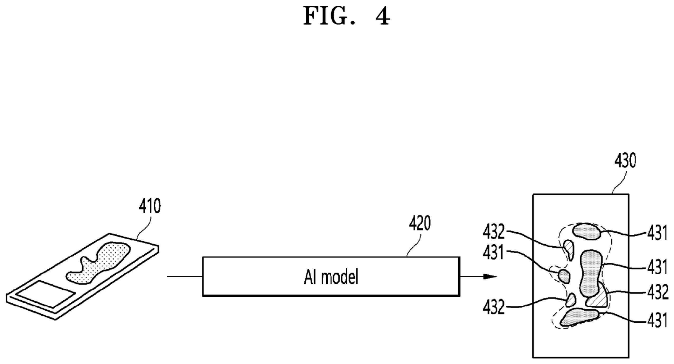

FIG. 4 is a view illustrating an example of identifying at least one tissue area from a pathological slide image by using an artificial intelligence model, according to an embodiment.

Referring to FIG. 4, in an embodiment, a processor may identify at least one tissue area 431 and 432 from a pathological slide image 430 by using an artificial intelligence model 420. In detail, the processor may identify the at least one tissue area 431 and 432 included in the pathological slide image 430 by inputting an image obtained by capturing a pathological slide 410 into the artificial intelligence model 420. The pathological slide image 430 may be an image obtained by capturing the pathological slide 410 that is fixed and stained via a series of chemical treatment processes for a tissue or the like removed from a human body.

The tissue refers to a collection of cells specialized to perform a common function, and a certain area in which the cells are clustered is referred to as a tissue area. In an embodiment, the at least one tissue area 431 and 432 may include a cancer area 431 and a stroma area 432.

The cancer area 431 refers to an area in which tumor cells showing invasion are clustered, and the stroma area 432 refers to a peripheral area of the cancer area 431 in which tumor-related stroma changes, such as desmoplasia or aggregation of lymphoid cells, are observed.

The artificial intelligence model 420 may be a machine learning model. As an example, the artificial intelligence model 420 may be trained to infer histological information for all or a portion of the pathological slide image 430 (e.g., at least one patch included in the pathological slide image 430). In this case, the histological information generated through an annotation task may be used to train the artificial intelligence model 420. For example, the histological information may include information (e.g., the number of particular cells and information regarding a tissue in which the particular cells are arranged) regarding a cell within a patch (e.g., a tumor cell, lymphocyte, macrophage, a dendritic cell, fibroblast, an endothelial cell, or the like), but is not limited thereto. As another example, the artificial intelligence model 420 may be trained to infer characteristics of at least one of a cell, a tissue, and a structure within the pathological slide image 430.

In an embodiment, the artificial intelligence model 420 may identify the at least one tissue area 431 and 432 by determining, by using the histological information, information regarding an area in which a cell is arranged in the pathological slide image 430.

Referring back to FIG. 3, in operation 320, the processor may generate spatial distribution-related information regarding the at least one tissue area, on the basis of an image arithmetic operation on the at least one tissue area.

As an example, the processor may identify a spatial distribution of the at least one tissue area on the basis of the image arithmetic operation on the at least one tissue area.

The spatial distribution of the at least one tissue area may include a shape and a fragment of the at least one tissue area, a boundary at which different tissue areas are in contact with each other, and the like. In other words, the spatial distribution of the at least one tissue area refers to how the at least one tissue area is arranged in the pathological slide image and a location relationship between different tissue areas.

In the present disclosure, the spatial distribution-related information may include information related to the spatial distribution of the tissue area acquired by the processor on the basis of the image arithmetic operation on the at least one tissue area included in the pathological slide image. The spatial distribution-related information may include at least one of area information of each of a plurality of sections included in the at least one tissue area based on a boundary at which the cancer area and the stroma area are in contact with each other, area information of an intersection area in which an area to which the stroma area is extended by a preset length overlaps the cancer area, the number of fragments of the cancer area included in the at least one tissue area, information regarding an analysis range set on the basis of the boundary at which the cancer area and the stroma area included in the at least one tissue area are in contact with each other, information (e.g., a location, an area, and the like) regarding the boundary at which the cancer area and the stroma area included in the at least one tissue area are in contact each other, location information of the cancer area, and location information of the stroma area.

The image arithmetic operation may include at least one of image filtering for processing an image by applying a particular kernel to an image, binarization for dividing pixel values of the image into two groups according to a threshold value, image transform for changing a size or shape of the image, image segmentation for segmenting the image into a plurality of portions, and a morphological operation for manipulating the shape of the image via dilation, erosion, or the like, but is not limited thereto. The processor may perform an arithmetic operation on each pixel with respect to the pathological slide image.

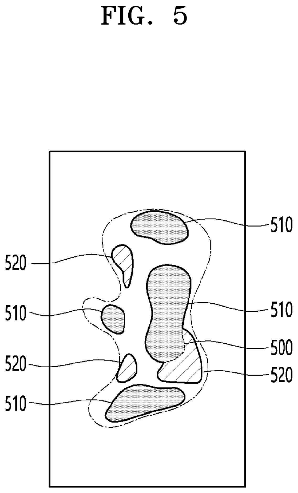

FIG. 5 is a view illustrating an example of a spatial distribution of a tissue area, according to an embodiment.

In an embodiment, a processor may identify a spatial distribution of at least one tissue area 510 and 520 on the basis of an image arithmetic operation on the at least one tissue area 510 and 520.

As an example, the spatial distribution may include fragments of a cancer area 510 and/or a stroma area 520. In other words, the processor may detect the fragments of the cancer area 510 and/or the stroma area 520 on the basis of the image arithmetic operation on the at least one tissue area 510 and 520. For example, the processor may perform the image arithmetic operation for determining whether or not the cancer area 510 includes separate areas (i.e., fragments) or attached areas by analyzing the identified cancer area 510 in preset units (e.g., pixels). In other words, in the case where the processor determines that the cancer areas 510 are separated by even one pixel, the processor may detect the cancer areas 510 as different fragments.

The processor may recognize the cancer area 510 as a fragment unit as a result of the image arithmetic operation, and generate spatial distribution-related information including the number of fragments of the cancer area 510. As illustrated in FIG. 5, the processor may detect four fragments of the cancer area 510 and generate spatial distribution-related information having a value of 4 as the number of fragments of the cancer area 510.

Meanwhile, when analyzing a pathological slide image, only basic numerical values, such as the entire area of the cancer area 510 and/or the stroma area 520, the number of tumor cells, the number of immune cells, a density of tumor cells, and a density of immune cells, are used as biomarkers, and thus, an issue is raised that the basic numerical values are limited in use for treatment responsiveness. However, according to the embodiment of the present disclosure described above, accuracy of predicting treatment responsiveness may be improved by presenting, as a new biomarker, an index reflecting a shape a degree of fragmentation, and the like in which the cancer area 510 and/or the stroma area 520 are distributed within the pathological slide image.

As another example, the spatial distribution may include a boundary (hereinafter, referred to as a tumor-stroma contact boundary) 500 at which the cancer area 510 and the stroma area 520 are in contact with each other. In other words, the processor may detect information related to the tumor-stroma contact boundary 500 and/or the vicinity of the tumor-stroma contact boundary 500 on the basis of the image arithmetic operation on the at least one tissue area 510 and 520.

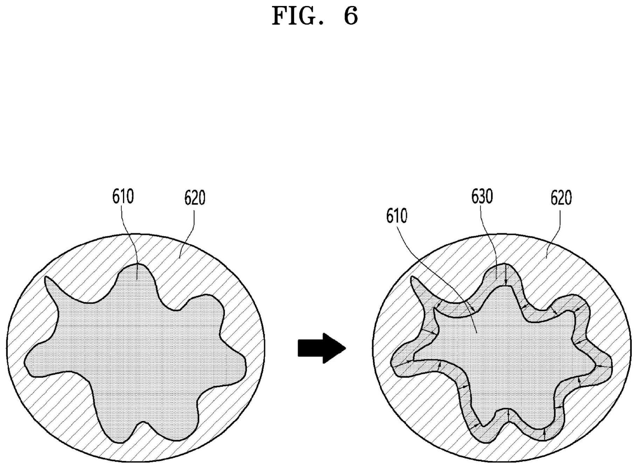

FIG. 6 is a view illustrating an example in which a cancer area and a stroma area are in contact with each other, according to an embodiment.

In the present disclosure, the case where "the cancer area 510 and the stroma area 520 are in contact with each other" includes all of a case where a contour of the cancer area 510 and a contour of the stroma area 520 are in contact with each other, a case where the cancer area 510 and the stroma area 520 do not overlap each other but are located within a preset adjacent distance, and the like.

Hereinafter, a process in which a processor performs an image arithmetic operation on at least one tissue area 610 and 620 to detect information related to a tumor-stroma contact boundary is described.

FIG. 6 illustrates an area to which a stroma area 620 is extended by a preset length. In an embodiment, the processor may set the area to which the stroma area 620 is extended by the preset length. Also, the processor may set an area (not shown) to which a cancer area 610 is contracted by a preset length. In detail, the processor may set an extended stroma area by extending (expanding) the stroma area 620 by the preset length and set a contracted area by contracting the cancer area 610 by the preset length. Here, the processor may extract a first intersection area between the extended stroma area and the cancer area 610. As an example, the processor may set an extended stroma area by extending the stroma area 620 by a first length (e.g., 10_{µm}) and set a contracted cancer area by contracting the cancer area 610 by the first length. Subsequently, the processor may extract an intersection of a difference between the expanded stroma area and the stroma area 620 and a difference between the cancer area 610 and the contracted cancer area, and determine the same as a first intersection area.

In another embodiment, the processor may set an area (not shown) to which the cancer area 610 is extended by a preset length. In addition, the processor may set an area (not shown) to which the stroma area 620 is contracted by a preset length. In detail, the processor may set an extended cancer area by extending (expanding) the cancer area 610 by a preset length and set a contracted stroma area by contracting the stroma area 620 by a preset length. Here, the processor may extract a first intersection area between the extended cancer area and the stroma area 620. As an example, the processor may set an extended cancer area by extending the cancer area 610 by a first length (e.g., 10 _{µm}) and set a contracted stroma area by contracting the stroma area 620 by the first length. Subsequently, the processor may extract an intersection of a difference between the extended cancer area and the cancer area 610 and a difference between the stroma area 620 and the contracted stroma area, and determine the same as the first intersection area.

Meanwhile, in an embodiment, the processor may determine area information regarding an area corresponding to one section from among a plurality of sections included in a cancer area, on the basis of the first intersection area. In another embodiment, on the basis of the first intersection area, the processor may calculate an evaluation index (e.g., a density of an analysis target cell in each of a plurality of sections generated on the basis of a tumor-stroma boundary, a tumor-stroma contact index, or the like) associated with treatment responsiveness.

The example in which the cancer area 610 and the stroma area 620 are extended or contracted by the preset length is described above by using FIG. 6, but the processor may extract a first intersection area and a second intersection area by extending or contracting the cancer area 610 and the stroma area 620 by a preset width or the preset number of particular units (e.g., the preset number of pixels).

Areas of a tissue adjacent to a tumor-stroma contact boundary are significant portions at which tumor infiltrating or invasion occurs, are closely related to tumor progression or metastasis, and thus need to be accurately detected. According to an embodiment of the present disclosure, an area of a tissue adjacent to a tumor-stroma contact boundary is detected on the basis of an image arithmetic operation on a pathological slide image, and thus, accuracy thereof may be improved.

Referring back to FIG. 3, in operation 330, the processor calculates an evaluation index associated with treatment responsiveness, on the basis of spatial distribution-related information.

Subsequently, the processor may calculate the evaluation index associated with the treatment responsiveness, on the basis of the spatial distribution-related information.

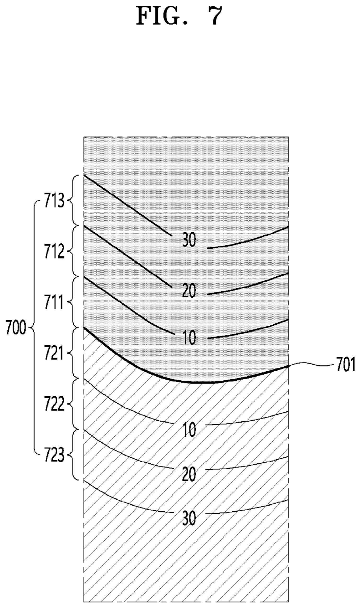

FIG. 7 is an example view of a plurality of sections according to an embodiment.

In an embodiment, a processor may segment a cancer area and a stroma area into a plurality of sections 711 to 713 and 721 to 723 on the basis of a preset interval, on the basis of a tumor-stroma contact boundary 701.

As an example, the preset interval may be 10_{µm}. The preset interval may be set on the basis of information of at least one of the greatest size of a cell, the greatest size of a target cell, an average size of the target cell, and an average size (e.g., 7_{µm} or 8_{µm}) of the cell, and the preset interval is not limited to 10_{µm} and may be changed according to various embodiments. For example, a preset interval in a direction of the stroma area and a preset interval in a direction of the cancer area may be set to different values.

In an embodiment, the processor may set an analysis range 700 in the direction of the stroma area and the direction of the cancer area, on the basis of the tumor-stroma contact boundary 701. For example, the analysis range 700 may refer to an area that is the sum of a range of 30_{µm} in the direction of the stroma area on the basis of the tumor-stroma contact boundary 701 and a range of 30_{µm} in the direction of the cancer area on the basis of the tumor-stroma contact boundary 701. However, the analysis range 700 is not limited to the embodiment described above. As a non-limiting example, the analysis range 700 may include the stroma area and the cancer area at different distances on the basis of the tumor-stroma contact boundary 701.

In an embodiment, the first intersection area described above with reference to FIG. 6 may be any one section from among the plurality of sections 711 to 713 and 721 to 723. In other words, a first intersection area in which an area to which the stroma area is extended by a first length overlaps the cancer area may be any one section from among the plurality of sections 711 to 713 and 721 to 723 in the direction of the cancer area. Similarly, a first intersection area in which an area to which the cancer area is extended by a first length overlaps the stroma area may be any one section from among the plurality of sections 711 to 713 and 721 to 723 in the direction of the stroma area.

In another embodiment, on the basis of an area to which the stroma area is extended by a second length (e.g., 20_{µm}) that is greater than the first length (e.g., 10_{µm}) described above and an area to which the cancer area is contracted by the second length, the processor may identify a second intersection area in which an area extended by the second length overlaps an area to which the cancer area is contracted by the second length. In detail, the processor may set an extended stroma area by extending the stroma area by the second length, and set a contracted cancer area by contracting the cancer area by the second length. Subsequently, the processor may extract an intersection of a difference between the stroma area extended by the second length and the stroma area extended by the first length and a difference between the cancer area contracted by the first length and the cancer area contracted by the second length, and determine the same as a second intersection area. The second intersection area determined as described above may be any one section from among the plurality of sections 711 to 713 and 721 to 723 in the direction of the cancer area.

Similarly, to determine any one section from among the plurality of sections 711 to 713 and 721 to 723 in the direction of the stroma area, on the basis of an area to which the cancer area is extended by the second length and an area to which the cancer area is contracted by the second length, the processor may identify a second intersection area in which an area extended by the second length overlaps an area to which the stroma area is contracted by the first length. The description of the same principle as the embodiment of detecting a section in the direction of the cancer area on the basis of the second length is omitted.

In an embodiment, the processor may calculate a density of at least one analysis target cell included in each of the plurality of sections 711 to 713 and 721 to 723. For example, an analysis target cell may be a tumor infiltrating lymphocyte. On the basis of an image arithmetic operation on at least one tissue area, the processor may determine an area of each of the plurality of sections 711 to 713 and 721 to 723 included in the at least one tissue area, on the basis of a boundary at which the cancer area and the stroma area are in contact with each other. The processor may determine the number of analysis target cells included in each of the plurality of sections 711 to 713 and 721 to 723, from among detected target cells. Alternatively, the processor may determine the number of analysis target cells included in a corresponding section, on the basis of information (a location, an area) regarding each of the plurality of sections 711 to 713 and 721 to 723. The processor may determine a density of analysis target cells in the corresponding section by using an area of each of the plurality of sections 711 to 713 and 721 to 723 and the number of analysis target cells included in the corresponding section.

In an embodiment, the processor may detect one or more grids including the tumor-stroma contact boundary 701, from among a plurality of grids of a pathological slide image. The one or more grids are defined as boundary grids.

Subsequently, the processor may calculate a density of an immune cell in each of the plurality of sections 711 to 713 and 721 to 723 included in the one or more boundary grids. In addition, the processor may predict treatment responsiveness of a patient from whom at least one tissue area is collected, to an immune anticancer agent, on the basis of the density of the immune cell. For example, the immune cell may be a tumor infiltrating lymphocyte.

Hereinafter, an embodiment in which the processor predicts the treatment responsiveness to the immune anticancer agent on the basis of the density of the immune cell is described.

The processor may determine at least one of an immune phenotype of at least one area within the pathological slide image and information associated with the immune phenotype, on the basis of at least one of a density of immune cells in the plurality of sections 711 to 713 in the direction of the cancer area and a density of immune cells in the plurality of sections 721 to 723 in the direction of the stroma area.

In detail, in the case where the density of immune cells in the plurality of sections 711 to 713 within the cancer area is greater than or equal to a first threshold density, the processor may determine an immune phenotype of at least a partial area within the pathological slide image as immune inflamed. Also, in the case where the density of the immune cells in the plurality of sections 711 to 713 within the cancer area is less than the first threshold density and a density of immune cells in the plurality of sections 721 to 723 within the stroma area is greater than or equal to a second threshold density, the processor may determine an immune phenotype of at least a partial area within the pathological slide image as immune excluded. In addition, in the case where the density of the immune cells in the plurality of sections 711 to 713 within the cancer area is less than the first threshold density and the density of the immune cells in the plurality of sections 721 to 723 within the stroma area is less than the second threshold density, the processor may determine an immune phenotype of at least a partial area within the pathological slide image as immune desert.

Here, the processor may determine the immune phenotype for each of the plurality of sections 711 to 713 and 721 to 723 within the pathological slide image. Accordingly, in the case where the immune phenotype the most included within the plurality of sections 711 to 713 and 721 to 723 is immune inflamed, the processor may predict that a patient associated with the corresponding pathological slide image responds to an immune anticancer agent (i.e., the patient is a responder). In contrast, in the case where the immune phenotype the most included within the plurality of sections 711 to 713 and 721 to 723 is immune excluded or immune desert, the processor may predict that the patient associated with the corresponding pathological slide image does not respond to the immune anticancer agent (i.e., the patient is a non-responder).

As described above, by segmenting the periphery of the tumor-stroma contact boundary 701 into the plurality of sections 711 to 713 and 721 to 723 and analyzing each section, the understanding of tumor progression may be improved, and an effective treatment strategy may be developed accordingly.

Meanwhile, immune cells in the section 711 inside 10_{µm} and the section 712 inside 20_{µm} in the direction of the cancer area on the basis of the tumor-stroma contact boundary 701 have a high association with treatment responsiveness (e.g., responsiveness to an immune anticancer agent). In an embodiment, the processor may set a weight to a density of at least one tumor infiltrating lymphocyte in the plurality of sections 711 to 713 and 721 to 723. As an example, a high weight may be set to a density of immune cells in the section 711 inside 10_{µm} and the section 712 inside the 20_{µm} in the direction of the cancer area, from among the plurality of sections 711 to 713 and 721 to 723. However, the example of setting weights to the plurality of sections 711 to 713 and 721 to 723 is not limited thereto.

In an embodiment, the processor may predict treatment responsiveness by reflecting the weight.

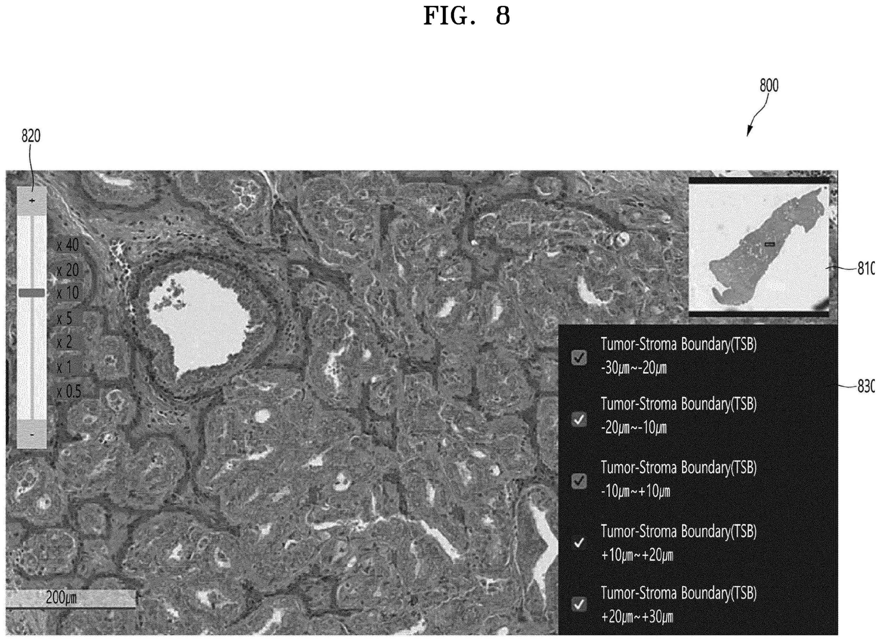

FIG. 8 is an example view of a pathological slide image in which a plurality of sections are displayed, according to an embodiment.

Referring to a pathological slide image 800 illustrated in FIG. 8, a first interface 810 indicating an area currently displayed in the pathological slide image 800, a second interface 820 capable of adjusting a resolution, and a third interface 830 describing a range of each of a plurality of sections are illustrated.

In an embodiment, a user may select, in the first interface 810, a portion of the pathological slide image 800 desired to identify. In addition, in the second interface 820, the user may adjust the resolution by using zoom in/out function for the pathological slide image 800. In the third interface 830, information regarding a plurality of sections as described above may be identified. A tumor-stroma boundary (TSB) illustrated in the drawing may correspond to a tumor-stroma contact boundary.

As described above, the processor may more deeply analyze the pathological slide image 800 by generating an interface for providing the user with a tumor-stroma contact boundary and a plurality of sections on the periphery of the tumor-stroma contact boundary.

In an embodiment, the plurality of sections may be expressed in different colors within the pathological slide image 800.

Referring back to FIG. 6, in an embodiment, the processor may determine an intersection area 630 in which an area to which the stroma area 620 is extended by a preset length overlaps the cancer area 610. An overlap between two areas refers to an area corresponding to an intersection of the two areas.

In an embodiment, the processor may calculate a tumor-stroma contact index on the basis of a ratio of an area of the intersection area 630 to an area of the cancer area 610. For example, the processor may calculate the tumor-stroma contact index on the basis of a ratio of an area of an intersection area in which an area to which the stroma area 620 is extended by a first length overlaps the cancer area 610, to the area of the cancer area 610.

A higher tumor-stroma contact index indicates that many portions at which the cancer area 610 and the stroma area 620 are in contact with each other are present, and at this time, responsiveness to an immune anticancer agent may be predicted to be high. Therefore, according to an embodiment of the present disclosure, a new biomarker may be presented by considering characteristics of not only the areas of the cancer area 610 and the stroma area 620, but also of shapes and distributions thereof. In addition, medical information such as a responsiveness to an immune anticancer agent derived from the same and a basis for the same may be identified together.

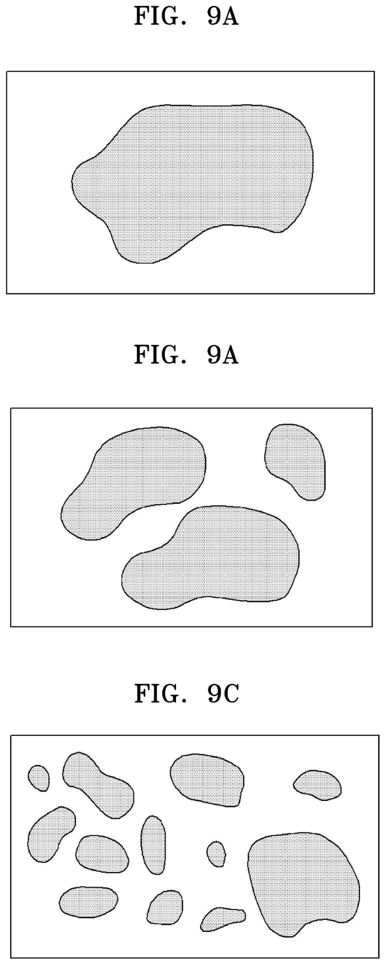

FIGS. 9A to 9C are views illustrating an example of calculating a fragmentation index, according to an embodiment.

An assumption is made that FIGS. 9A to 9C illustrate a cancer area having the same area. Here, the numbers of fragments of the cancer area in FIGS. 9A to 9C may be 1, 3, and 12, respectively.

In an embodiment, a processor may calculate a fragmentation index on the basis of a ratio of the number of fragments to an area of a cancer area. In other words, the fragmentation index refers to the average number of fragments present per unit area.

Comparing FIGS. 9A to 9C, the cancer area in FIG. 9A includes one mass and has the lowest fragmentation index. In addition, the cancer area in FIG. 9B includes three fragments and has a fragmentation index higher than the cancer area in FIG. 9A. The cancer area in FIG. 9C includes 12 fragments and has the highest fragmentation index.

In an embodiment, the processor may compare an area of each of a plurality of fragments constituting a cancer area with a preset reference area (e.g., 968.2_{µm}²). The processor may determine the number of fragments in the cancer area by identifying the number of at least one fragment having an area greater than or equal to the preset reference area.

In an embodiment, the processor may determine whether or not each of a plurality of fragments constituting the cancer area contacts a stroma area. The processor may determine the number of fragments in the cancer area by identifying the number of at least one fragment that is in contact with the stroma area. For example, the processor may determine the number of fragments in the cancer area by identifying the number of at least one fragment that is in contact with an outline of the stroma area within a pathological slide image. The processor may determine the number of fragments in the cancer area by identifying the number of at least one fragment that does not overlap the stroma area but is located within a preset adjacent distance within the pathological slide image. As an example, the processor may use whether or not the first intersection area described above is present, to identify the number of at least one fragment located within a preset adjacent distance to the stroma area, within the pathological slide image. In other words, the processor may determine the cancer area which does not overlap the stroma area but includes the first intersection area, as a fragment located within the preset adjacent distance.

The fragmentation index reflects relative growth rates of the cancer area and the stroma area, which may more precisely predict treatment responsiveness of cancer patients to an immune anticancer agent. As illustrated in FIG. 9A, the cancer area including one mass may be interpreted as having a faster growth rate than the cancer areas in FIGS. 9B and 9C.

The processor may easily identify the relative growth rate of the cancer area to the stroma area and an effect of the stroma area on the cancer area, by calculating the fragmentation index.

According to the embodiments of the present disclosure described above, predicting treatment responsiveness to an immune anticancer agent in advance may help an expert prescribe more appropriate and efficient customized treatment for patients. In addition, the prediction may ultimately contribute to an increase in a survival rate of a patient.

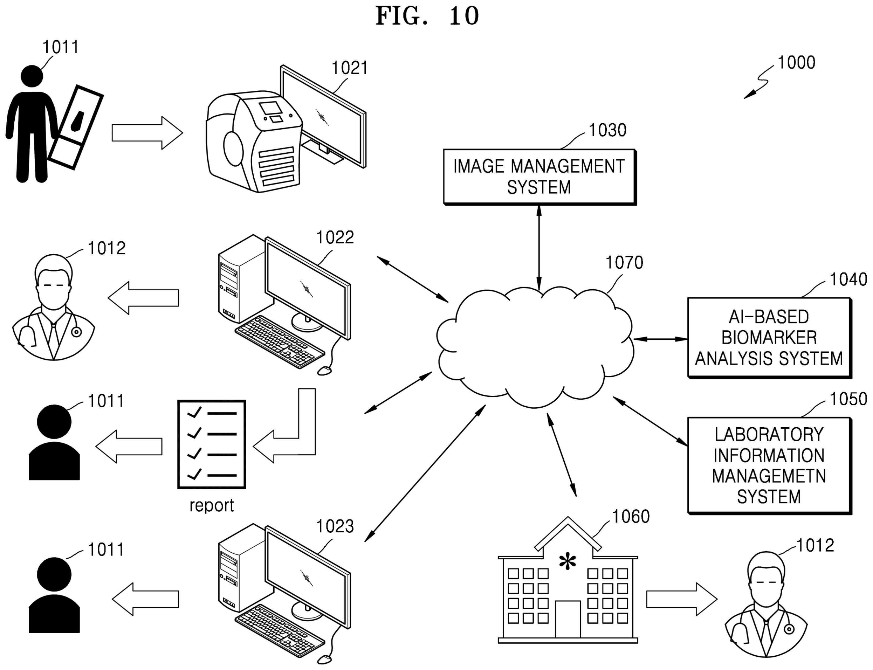

FIG. 10 is a view illustrating another example of a system for analyzing a pathological slide image.

Referring to FIG. 10, a system 1000 refers to an example of a system and a network for providing, processing, and reviewing slide images of tissue specimens by using an artificial intelligence model.

According to various embodiments of the present disclosure, the method described above with reference to FIGS. 2A to 10 may be performed by at least one or a combination of user terminals 1022 and 1023, an image management system 1030, an Al-based biomarker analysis system 1040, a laboratory information management system 1050, and a hospital or laboratory server 1060.

A scanner 1021 may acquire a digitized image from a tissue sample slide generated by using a tissue sample of a subject 1011. For example, each of the scanner 1021, the user terminals 1022 and 1023, the image management system 1030, the Al-based biomarker analysis system 1040, the laboratory information management system 1050, and/or the hospital or laboratory server 1060 may be connected to a network 1070, such as the Internet, via one or more computers, servers, and/or mobile devices or may communicate with a user 1012 via one or more computers, and/or mobile devices.

The user terminals 1022 and 1023, the image management system 1030, the Al-based biomarker analysis system 1040, the laboratory information management system 1050, and/or the hospital or laboratory server 1060 may generate tissue samples of one or more subjects 1011, tissue sample slides (pathological sides), digitized images of the tissue sample slides (the pathological slides), or any combination thereof, or otherwise, acquire the same from another apparatus. In addition, the user terminals 1022 and 1023, the image management system 1030, the Al-based biomarker analysis system 1040, the laboratory information management system 1050, and/or the hospital or laboratory server 1060 may acquire any combination of subject-specific information, such as age, medical history, cancer treatment history, family history, past biopsy records of the subject 1011, or disease information of the subject 1011.

The scanner 1021, the user terminals 1022 and 1023, the Al-based biomarker analysis system 1040, the laboratory information management system 1050, and/or the hospital or laboratory server 1060 may transmit a digitized pathological slide image, subject-specific information, and/or the result of analyzing the digitized pathological slide image to the image management system 1030 through the network 1070. The image management system 1030 may include a repository for storing a received image and a repository for storing a result of analysis.

In addition, according to various embodiments of the present disclosure, an artificial intelligence model, which is learned and trained to predict, from a pathological slide image of the subject 1011, at least one of information regarding at least one cell, information regarding at least one area, information related to a biomarker, medical diagnosis information, and/or medical treatment information, may be stored in the user terminals 1022 and 1023, the image management system 1030, the Al-based biomarker analysis system 1040, and the like and operate.

As described above, a computer apparatus according to an embodiment of the present disclosure may extract a spatial distribution of a tissue area from a pathological slide image and automatically calculate an evaluation index from the spatial distribution. Therefore, an analysis method by which a pathologist directly examines a portion of a distribution of a tissue in the whole pathological slide image may be replaced. In addition, prediction accuracy and analysis speed of treatment responsiveness of a patient to an immune anticancer agent may be improved.

Meanwhile, the above-described method may be written as a program that may be executed on a computer, and may be implemented in a general-purpose digital computer that operates the program by using a computer-readable recording medium. In addition, a structure of data used in the above-described method may be recorded in a computer-readable recording medium via various types of means. The computer-readable recording medium includes a storage medium, such as a magnetic storage medium (e.g., ROM, RAM, a USB, a floppy disk, a hard disk, or the like) and an optical reading medium (e.g., CD-ROM, DVD, or the like).

Those skilled in the art related to the present embodiment may understand that the present embodiment may be implemented in a modified form within the scope that does not depart from the essential characteristics of the above description. Therefore, the disclosed methods should be considered in an illustrative rather than a restrictive sense, and the scope of the present disclosure should be defined by claims rather than by the foregoing description, and should be construed to include all differences within the scope equivalent thereto.

## Claims

1. A computing apparatus comprising:
at least one memory; and
at least one processor, wherein the at least one processor is configured to identify at least one tissue area from a pathological slide image by using an artificial intelligence model, generate spatial distribution-related information regarding the at least one tissue area on the basis of an image arithmetic operation on the at least one tissue area, and calculate an evaluation index associated with treatment responsiveness, on the basis of the spatial distribution-related information.

2. The computing apparatus of claim 1, wherein the spatial distribution-related information comprises at least one of area information of each of a plurality of sections included in the at least one tissue area based on a boundary at which a cancer area and a stroma area are in contact with each other, area information of an intersection area in which an area to which the stroma area is extended by a preset length overlaps the cancer area, and a number of fragments in the cancer area included in the at least one tissue area.

3. The computing apparatus of claim 1, wherein the at least one processor is further configured to segment the cancer area and the stroma area into a plurality of sections on the basis of a preset interval, on the basis of a boundary at which the cancer area and the stroma area included in the at least one tissue area are in contact with each other, and calculate a density of at least one analysis target cell included in each of the plurality of sections.

4. The computing apparatus of claim 3, wherein the at least one processor is further configured to detect one or more boundary grids comprising the boundary, from among a plurality of grids of the pathological slide image, and calculate a density of a tumor infiltrating lymphocyte in each of the plurality of sections included in the one or more boundary grids.

5. The computing apparatus of claim 4, wherein the at least one processor is further configured to set a weight to a density of at least one analysis target cell in at least one section from among the plurality of sections.

6. The computing apparatus of claim 3, wherein the at least one processor is further configured to: on the basis of an extended area to which the stroma area is extended by a first length and an area to which the cancer area is contracted by the first length, identify a first intersection area in which the extended area overlaps the cancer area; and determine area information regarding an area corresponding to one section from among a plurality of sections included in the cancer area, on the basis of the first intersection area.

7. The computing apparatus of claim 6, wherein the at least one processor is further configured to: on the basis of an area to which the stroma area is extended by a second length greater than the first length and an area to which the cancer area is contracted by the second length, identify a second intersection area in which the area extended by the second length overlaps an area to which the cancer area is contracted by a first length; and determine area information regarding an area corresponding to another section from among the plurality of sections included in the cancer area, on the basis of the second intersection.

8. The computing apparatus of claim 3, wherein the at least one processor is further configured to: on the basis of an extended area to which the cancer area is extended by a first length and an area to which the stroma area is contracted by the first length, set an intersection area in which the extended area overlaps the stroma area; and determine area information regarding an area corresponding to one section from among a plurality of sections included in the stroma area, on the basis of the intersection area.

9. The computing apparatus of claim 1, wherein the at least one processor is further configured to determine an intersection area in which an area to which a stroma area is extended by a preset length overlaps a cancer area, and calculate a tumor-stroma contact index on the basis of a ratio of an area of the intersection area to an area of the cancer area.

10. The computing apparatus of claim 1, wherein the at least one processor is further configured to detect fragments in a cancer area included in the at least one tissue area, and calculate a fragmentation index on the basis of a ratio of a number of fragments to an area of the cancer area.

11. A method of analyzing a pathological slide image, the method comprising:
identifying at least one tissue area from a pathological slide image by using an artificial intelligence model;
generating spatial distribution-related information regarding the at least one tissue area, on the basis of an image arithmetic operation on the at least one tissue area; and
calculating an evaluation index associated with treatment responsiveness, on the basis of the spatial distribution-related information.

12. The method of claim 11, wherein the spatial distribution-related information comprises at least one of area information of each of a plurality of sections included in the at least one tissue area based on a boundary at which a cancer area and a stroma area are in contact with each other, area information of an intersection area in which an area to which the stroma area is extended by a preset length overlaps the cancer area, and a number of fragments in the cancer area included in the at least one tissue area.

13. The method of claim 11, wherein the calculating comprises:
segmenting the cancer area and the stroma area into a plurality of sections on the basis of a preset interval, on the basis of a boundary at which the cancer area and the stroma area included in the at least one tissue area are in contact with each other; and
calculating a density of at least one analysis target cell included in each of the plurality of sections.

14. The method of claim 13, wherein the calculating the evaluation index comprises:
detecting one or more boundary grids comprising the boundary, from among a plurality of grids of the pathological slide image; and
calculating a density of a tumor infiltrating lymphocyte in each of the plurality of sections included in the one or more boundary grids.

15. The method of claim 14, wherein the calculating the density of the tumor infiltrating lymphocyte comprises setting a weight to a density of at least one analysis target cell in at least one section from among the plurality of sections.

16. The method of claim 13, wherein the generating comprises:
on the basis of an extended area to which the stroma area is extended by a first length and an area to which the cancer area is contracted by the first length, identifying an intersection area in which the extended area overlaps the cancer area; and
determining area information regarding an area corresponding to one section from among a plurality of sections included in the cancer area, on the basis of the intersection area.

17. The method of claim 13, wherein the generating comprises:
on the basis of an extended area to which the cancer area is extended by a first length and an area to which the stroma area is contracted by the first length, setting an intersection area in which the extended area overlaps the stroma area; and
determining area information regarding an area corresponding to one section from among a plurality of sections included in the cancer area, on the basis of the intersection area.

18. The method of claim 11, wherein the calculating comprises:
determine an intersection area in which an area to which a stroma area is extended by a preset length overlaps a cancer area; and calculating a tumor-stroma contact index on the basis of a ratio of an area of the intersection area to an area of the cancer area.

19. The method of claim 11, wherein the generating comprises
detecting a fragment of a cancer area included in the at least one tissue area, and
the calculating comprises calculating a fragmentation index on the basis of a ratio of a number of the fragments to an area of the cancer area.

20. A computer-readable recording medium having recorded thereon a program for causing a computer to execute the method of claim 11.
